# EUROPEAN PATENT SPECIFICATION

(11) **EP 1 804 060 B1**
(45) Date of publication and mention of the grant of the patent: **18.08.2010**
(21) Application number: 06120263.6
(22) Date of filing: 07.09.2006
(51) Int. Cl.: G01N 33/50

(54) **Method and apparatus for detecting presence of toxic material within sample**
Verfahren und Vorrichtung für den Nachweis von toxischem Material in einer Probe
Procédé et appareil pour détecter la présence de matériaux toxiques dans un échantillon

(30) Priority: 28.12.2005 KR 20050131881
(43) Date of publication of application: 04.07.2007
(73) Proprietor: Samsung Electronics Co., Ltd., Suwon-si, Gyeonggi-do (KR)
(72) Inventor: Han, Jung-im, Giheung-gu, Yongin-si Gyeonggi-do (KR); Choi, Soo-hyung, Banwol-dong, Hwaseong-si Gyeonggi-do (KR)
(74) Representative: Grünecker, Kinkeldey, Stockmair & Schwanhäusser Anwaltssozietät

(56) References cited:
- EP-A2- 0 228 290
- US-A1- 2003 059 839
- KNOBELOCH L M ET AL: "Use of submitochondrial particles for prediction of chemical toxicity in man" BULLETIN OF ENVIRONMENTAL CONTAMINATION AND TOXICOLOGY, SPRINGER VERLAG, NEW YORK, NY, US, vol. 44, no. 5, May 1990 (1990-05), pages 661-668, XP009075526 ISSN: 0007-4861

## Description

### BACKGROUND OF THE INVENTION

### 1. Field of the Invention

The present invention relates to a method and a kit for detecting the presence of toxic materials within a sample.

### 2. Description of the Related Art

Conventionally, use of mitochondria or sub-mitochondria particles is well known as a biological analysis instrument for detecting the presence of toxic materials within environmental samples. For example, U.S Patent No. 4,808,517 discloses (a) suspension of sub-mitochondria particles having competent mitochondrial enzyme formed from the inner membrane of mitochondria, (b) an analysis medium including a substrate in which either substrate or enzyme reaction product thereof can be detected using a spectroscopic detection method when the analysis medium is transformed by mitochondrial enzyme, and (c) a process of mixing environmental samples, which will be tested, in a common vessel and a method of analyzing toxic materials within an environmental sample including a process of determining how the sample influences upon enzyme activity within the suspension of sub-mitochondria particles by measuring the changes of the substrate using spectroscopic measurement.

EP 0228290 and Knobeloch et al., "Use of submitochondrial particles for prediction of chemical toxicity in man", Bulletin of Environmental Contamination and Toxicology, Springer Verlag, New York, NY, US, vol. 44, no. 5, 1990, 661-668, also disclose methods for testing the presence of toxic samples via spectrochemical analysis.

In general, tris-bipyridyl-ruthenium (Ru(bpy)₃(II)) complex is used to induce electrochemiluminescence (ECL) using an electrochemiluminescenet label (For example, U.S Patent Publication No. 2003/0059839). ECL is light emission caused by response of electrically stimulated species. A species which is induced to emit ECL is called an ECL label or an ECL active species.

As described above, conventional methods are mostly used for detecting optical signals, and methods of analyzing toxic materials by detecting electrical signals such as voltage or current are still unknown.

### SUMMARY OF THE INVENTION

The present invention provides an effective method of detecting the presence of toxic materials within a sample.

According to an aspect of the present invention, there is provided a method of detecting the presence of toxic materials within a sample, the method including: contacting sub-mitochondria particles having competent mitochondrial enzyme formed from the inner membrane of mitochondria, an electron donor which transmits electrons to the electron transfer system of sub-mitochondria particles, and a sample which will be tested ; adding tris-2,2'-bipyridyl-ruthenium (II) or (Ru(bpy)₃(II)) ion or [Ru(bpy)₃]²⁺ to the reaction mixture; and measuring electrical variables of the reaction mixture.

According to another aspect of the present invention, there is provided a kit for electrically analyzing the presence of toxic materials within a sample, the kit including: sub-mitochondria particles having competent mitochondrial enzyme formed from the inner membrane of mitochondria; an electron donor which transmits electrons to the electron transfer system of sub-mitochondria particles; and tris-2,2'-bipyridyl-ruthenium (II) or (Ru(bpy)₃(II)) ion or [Ru(bpy)₃]²⁺.

### BRIEF DESCRIPTION OF THE DRAWINGS

The above and other features and advantages of the present invention will become more apparent by describing in detail exemplary embodiments thereof with reference to the attached drawings in which:
FIG. 1A is a diagram of a current measuring apparatus according to an embodiment of the present invention and FIG. 1B is a schematic diagram of the current measuring apparatus;
FIG. 2 is a current density-voltage graph of a 7.25 mM NADH solution (Journal of Electroanalytical Chemistry 568 (2004) 301-313, FIG 3.);
FIG. 3 is a graph showing a cyclic voltammogram of 2.5 mM Ru(bpy)₃²⁺ in phosphate-buffered saline (PBS);
FIG. 4 is a graph illustrating a cathode peak current at 1.019 V in a cyclic voltammogram with respect to a concentration of ethanol;
FIG. 5 is a graph illustrating a cathode peak current at 1.019 V in a cyclic voltammogram with respect to a concentration of sorbitol; and
FIG. 6 is a graph illustrating a relationship between the value of a cathode peak current measured electrically and a NADH concentration measured optically.

### DETAILED DESCRIPTION OF THE INVENTION

Hereinafter, the present invention will be described more fully.

In an embodiment of the present invention, a method of detecting the presence of toxic materials within a sample according to an embodiment of the present invention includes contacting sub-mitochondria particles having competent mitochondrial enzyme formed from the inner membrane of mitochondria, an electron donor which transmits electrons to the electron transfer system of sub-mitochondria particles, and a sample which will be tested.

The sub-mitochondria particle (SMP) of the present invention is a lipid bilayer membrane vesicle obtained by the micell formation from a fraction of crista membrane when mitochondria are ruptured. That is, whole mitochondria derived from arbitrary origin is ruptured by ultrasonic waves, a detergent such as a digitonin or a French press , membrane fractions are separated from cytoplasmic residue by a centrifugal separation, and the membrane segments are then allowed to reform into vesicles which model the behavior of the intact inner membrane of mitochondria.

Such sub-mitochondria particle can model behavior of mitochondria and a large amount of sub-mitochondria particles also can be saved by being frozen or lyophilized after manufacturing and thus, the aliquot of a sub-mitochondria particle product can be easily used in performing a toxicant assay which takes a long time.

The sub-mitochondria particles may be manufactured by sonicating whole mitochondria for crista membrane to reform into micelle.

The sub-mitochondria particles used in the present invention can be manufactured using any conventional method or purchased commercially.

A method of manufacturing the sub-mitochondria particles is, for example, disclosed in U. S Patent No. 4,808,517 , incorporated by reference herein in its entirety. Firstly, in order to manufacture the sub-mitochondria particles, whole mitochondria should be prepared. Here, mitochondria derived from any arbitrary origin may be used. The sub-mitochondria particles themselves may be manufactured from fresh mitochondria or from frozen mitochondria. Once the sub-mitochondria particles are manufactured, they can be stored in a preserved mixture at -20 °C. Also, for storage purposes, the sub-mitochondria particles can be lyophilized. In order to use the lyophilized sub-mitochondria particles in the present invention, they can simply be defrosted. Lyophilized product can be saturated with an analysis medium before use and thus, can be simply reformed.

The sub-mitochondria particles include competent mitochondrial enzyme. In the term "competent mitochondrial enzyme" of the present invention, the enzyme involved in the electron transfer system of mitochondria is active. Accordingly, the method of an embodiment of the present invention refers to measuring how toxic materials affect the electron transfer system of mitochondria or to the electron transfer system of sub-mitochondria particles by changing to an electric signal. In more detail, toxic materials affect the enzyme of the electron transfer system of sub-mitochondria particles and thus, affect the amount of electrons transmitted from an electron donor to the electron transfer system. Subsequently, toxic materials affect the amount of electron donors within a reaction solution. The method of the present invention refers to electrically measuring of the amount of the electron donors within a reaction solution, thereby measuring the influence on the electron transfer system, that is, the extent of toxicity. However, the present invention is not limited thereto.

The mitochondrial enzyme may be consisted of NADH-dehydrogenase, coenzyme-Q-cytochrome C reductase, and cytochrome C oxidase.

The electron donor may include at least one selected from the group consisting of NADH and NADPH.

The toxic materials may be any toxic materials which have an influence on the electron transfer system of mitochondria or sub-mitochondria particles, for example, ethanol, methanol, phenols, heavy metals, and solvents, but are not limited thereto.

The method of contacting described above can be performed in any condition as long as the enzymes of the electron transfer system can react, for example, a physiological condition or a condition similar to a physiological condition. That is, the process of contacting may be performed in a solution having physiological salt and ionic strength near to pH of 7 (for example, a PBS solution) or in a buffer for a predetermined time. When the contacting method is performed under the physiological condition or in the solution similar thereto, the amount of the sub-mitochondria particles may be 0.1 to 0.5 mg/ml and the amount of the electron donors may be 1 to 30 mM, but the sub-mitochondria particles and the electron donors are not limited to these amounts. The concentration of the sub-mitochondria and the electron donors increase, the reaction speed also increases and thus, the sub-mitochondria and the electron donors can be used by considering the desired reaction speed and adjusting concentration appropriately.

In addition, the method of detecting the presence of toxic materials within a sample according to an embodiment of the present invention further includes adding tris-2,2'-bipyridyl-ruthenium (II) or (Ru(bpy)₃(II)) ion or [Ru(bpy)₃]²⁺ to the reaction mixture. Tris-2,2'-bipyridyl-ruthenium (II) or (Ru(bpy)₃(II)) ion or [Ru(bpy)₃]²⁺can be purchased commercially and may be used in a range of 1 to 5 mM. Since an electrical property of tris-2,2'-bipyridyl-ruthenium (II) or (Ru(bpy)₃(II)) ion or [Ru(bpy)₃]²⁺ may vary according to the concentration of the electron donors, tris-2,2'-bipyridyl-ruthenium (II) or (Ru(bpy)₃(II)) ion or [Ru(bpy)₃]²⁺ is added to measure electrical variables according to the concentration of the electron donor. More specifically, tris-2,2'-bipyridyl-ruthenium (II) or (Ru(bpy)₃(II)) ion or [Ru(bpy)₃]²⁺ is converted reversibly to + bivalence or + trivalence by an oxidation-reduction reaction, when voltage is applied thereto. For example, the extent of reversible oxidation-reduction is changed according to a concentration of NADH and an electrical signal of the solution including tris-2,2'-bipyridyl-ruthenium (II) or (Ru(bpy)₃(II)) ion or [Ru(bpy)₃]²⁺, for example, a current value at a specific voltage, is changed showing a linearity according to a concentration of NADH.

Moreover, the method of detecting the presence of toxic materials within a sample according to an embodiment of the present invention further includes measuring electrical variables of the reaction mixture. The electrical variables may include at least one selected from the group consisting of current, voltage, impedance, and capacitance.

More preferably, the electrical variables are one of a cathode peak current and an anode peak current at cyclic voltammogram. In this case, the electrical variables may be measured in a potential range that oxidized electron donor, NAD+ or NDAP+ is not reduced. The current may be measured in a potential range of 0.85 to 1.35 V. When the electrical variables include a current at a specific voltage, the measured current value increases compared to that of a control sample in which the tested compound is not included and it is determined that toxicity exists.

In another embodiment of the present invention, a kit for electrically analyzing the presence of toxic materials within a sample includes sub-mitochondria particles having competent mitochondrial enzyme formed from the inner membrane of mitochondria, an electron donor which transmits electrons to the electron transfer system of the sub-mitochondria particles, and tris-2,2'-bipyridyl-ruthenium (II) or (Ru(bpy)₃(II)) ion or [Ru(bpy)₃]²⁺.

The sub-mitochondria particles may be manufactured by sonicating whole mitochondria for crista membrane to reform into micelle.

The sub-mitochondria particle may be frozen or lyophilized.

The mitochondrial enzyme may include at least one enzyme selected from the group consisting of NADH-dehydrogenase, coenzyme-Q-cytochrome C reductase, and cytochrome C oxidase.

The electron donor may include at least one selected from the group consisting of NADH and NADPH.

In the kit of the present invention, the sub-mitochondria particles, the competent enzymes, the electron donors, toxic materials, and tris-2,2'-bipyridyl-ruthenium (II) or (Ru(bpy)₃(II)) ion or [Ru(bpy)₃]²⁺are described as in the method of the present invention.

The present invention will be described in greater detail with reference to the following examples. The following examples are for illustrative purposes and are not intended to limit the scope of the invention.

### Examples

### Structure of current measuring apparatus using a cyclic voltammetry method and range of applied voltage

### (1) Current measuring apparatus using a cyclic voltammetry method

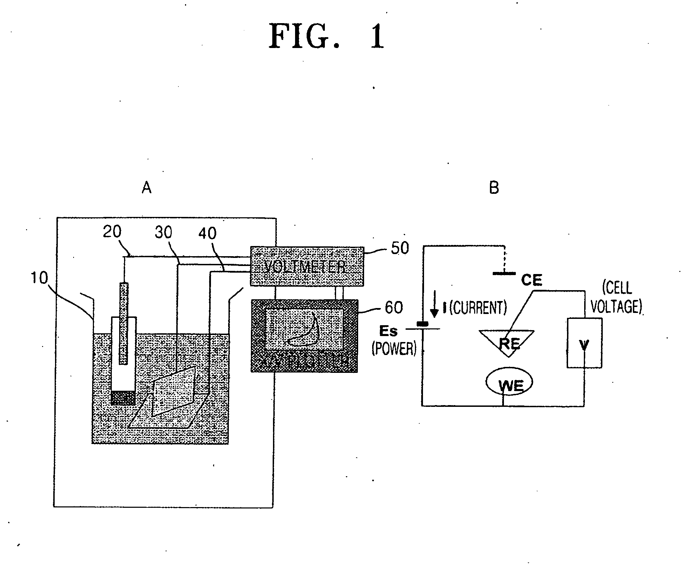

FIGS. 1A is a diagram of a current measuring apparatus according to an embodiment of the present invention and FIG. 1B is a schematic diagram of the current measuring apparatus. Referring to FIG. 1, a reaction vessel 10 includes a platinum standard electrode 20, a platinum working electrode 30, and an Ag/AgCl counter electrode 40. The platinum standard electrode 20, the platinum working electrode 30, and the Ag/AgCl counter electrode 40 are connected to a voltmeter 50 (PerkinElmer VMP multichannel potentiostat). An X/V plotter 60 is connected to the voltmeter 50.

In an Example of the present invention, an experiment was conducted by adding 6 mM of NADH (reduced nicotinamide adenine dinucleotide) which exists in 0.5XPBS into a reaction vessel and measuring a current range with a scan speed of 50 mV/sec CV and 7 ml of a measuring solution.

### (2) Range of applied voltage

In order to measure a peak current using a cyclic voltammetry method, voltage may be in a range where oxidized electron donors may not be reduced again. Accordingly, NAD+ or NADP+, which is an oxidized form of electron donor used in the Example may not be reduced to NADPH.

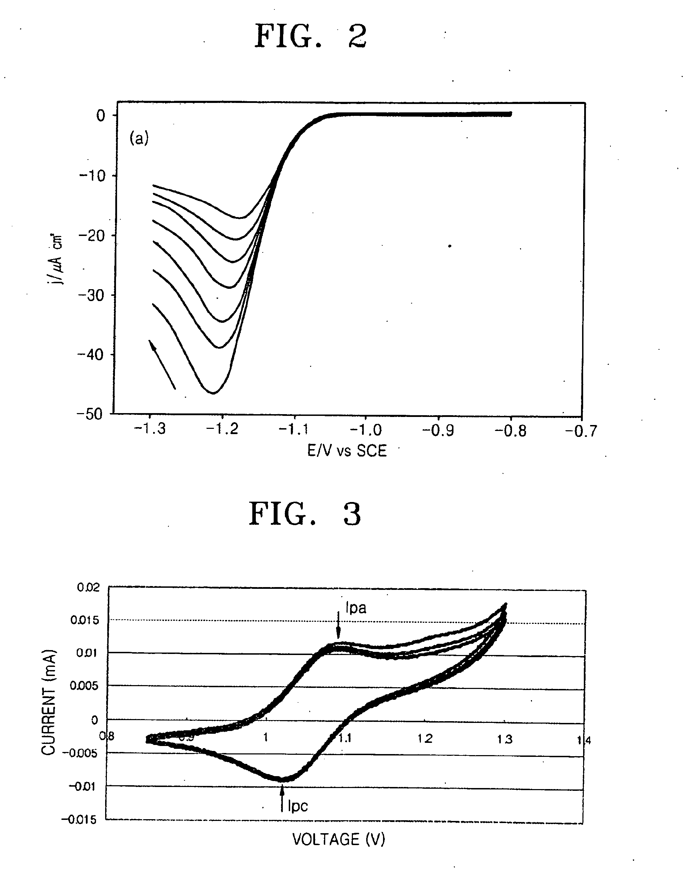

FIG. 2 is a conventional current density-voltage graph of a 7.25 mM NADH solution (Journal of Electroanalytical Chemistry 568 (2004) 301-313). Referring to FIG. 2, it can be seen that NAD+ is not reduced to NADH in a range of 0.85 to 1.35 V/Ag/AgCl. In the Examples described below, a peak current was measured in the range of 0.85 to 1.35 V/Ag/AgCl.

### Example 1: Detecting toxicity of ethanol and sorbitol according to the method of the present invention

A cathode peak current according to was measured using the current measuring apparatus illustrated in FIG. 1A, in accordance with the concentration of ethanol as a toxic material, and sorbitol as a non-toxic material against a cell.

0.5xPBS including 0.5 mg/ml of SMP (submitochondrial particle) (Havard Bioscience), 6 mM of NADH, and 50 mM of KCI was allowed to react at ambient temperature for 1 hour. Then the reaction solution and 5 mM Ru(bpy)₃²⁺ which exists in 0.5xPBS were mixed in a ratio of 1:1 to obtain a cyclic voltammogram using a voltmeter (potentiostat), thereby obtaining a cathode peak current. The cyclic voltammogram was measured with a scan speed of 50 mV/sec CV at a sweep range of 0.85 to 1.35 V. Subsequently, in the cyclic voltammogram of 2.5mM Ru(bpy)₃²⁺ in which toxic materials are not included, voltage generated at the cathode peak current and the anode peak current was 1.019 V and 1.2 V, respectively. FIG. 3 is a graph showing the cyclic voltammogram of 2.5mM Ru(bpy)₃²⁺ in phosphate-buffered saline (PBS).

Then, ethanol having various final concentration of 0%, 2.5%, 5%, and 10% or 2.5 % of sorbitol was added to 0.5xPBS including 0.5mg/ml of SMP (submitochondrial particle), 6 mM of NADH, and 50 mM of KCI to prepare 7 ml of a final reaction solution and was allowed to react for 1 hour at ambient temperature.

Next, absorbance of the final reaction solution was measured at 340 nm using a spectrophotometer and the concentration of NADH was measured using an optical method. In addition, the reaction solution and 5mM Ru(bpy)₃²⁺ which exists in 0.5xPBS were mixed in a ratio of 1:1 and a cathode peak current at 1.019 V was obtained using a voltmeter (potentiostat). The cyclic voltammogram was measured with a scan speed of 50 mV/sec CV at a sweep range of 0.85 to 1.35V.

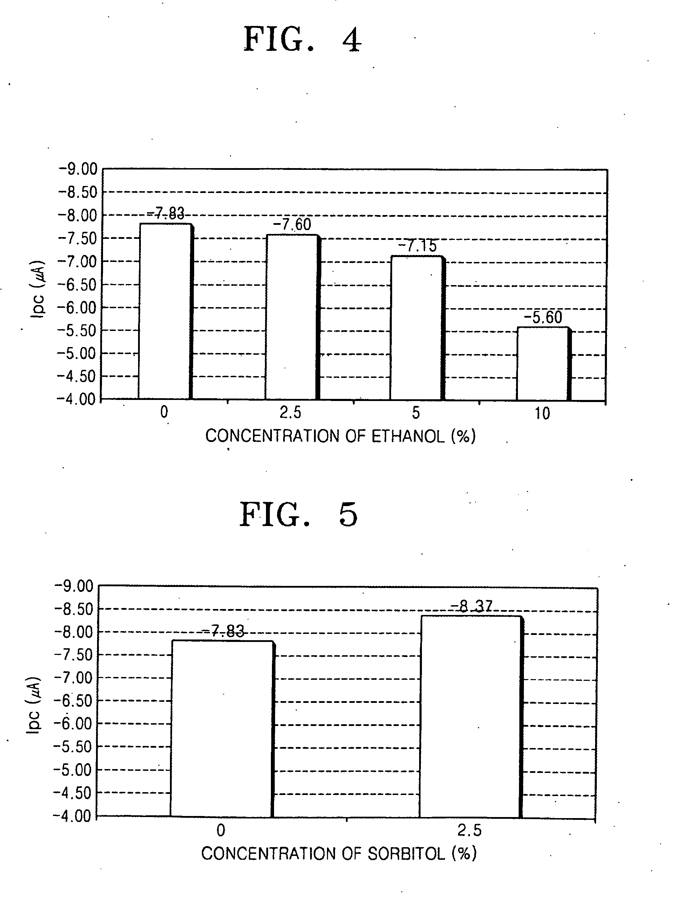

FIG. 4 is a graph illustrating a cathode peak current at 1.019V in a cyclic voltammogram with respect to a concentration of ethanol. Referring to FIG. 4, the higher a concentration of ethanol, the higher a cathode peak current, thereby increasing a concentration of NADH. That is, ethanol obstructs electron transfer reaction of sub-mitochondria particles indicating that a cell contains toxic material. In addition, in an ethanol concentration of 2.5 %, which is generally known as a concentration containing low amounts of toxic material, a cathode peak current was higher than in an ethanol concentration of 0 %, indicating high sensitivity of an electrical measuring method according to an embodiment of the present invention.

FIG. 5 is a graph illustrating a cathode peak current at 1.019V in a cyclic voltammogram with respect to a concentration of sorbitol. Referring to FIG. 5, when the concentration of sorbitol is 2.5 %, the cathode peak current is even lower than when the concentration of sorbitol is 0 % and it can be seen that sorbitol has no toxic material but slightly changes an electrical property of the solution including 5mM Ru(bpy)₃²⁺. The fact that sorbitol having a concentration of 2.5 % has no toxicity was identified by the measuring absorbance at 340 nm using a spectrophotometer and resulted in no changes compared to when sorbitol has a concentration of 0 % (data is not illustrated herein).

### Example 2 : Relationship between method according to the present invention and conventional optical method for detecting toxic materials

According to the method described in Example 1, a cathode peak current at 1.019V with respect to a concentration of ethanol was measured and a concentration of NADH was also measured by measuring absorbance at 340 nm using a spectrophotometer. Subsequently, a relationship between the value of a cathode peak current and a concentration of NADH measured using an optical method was identified.

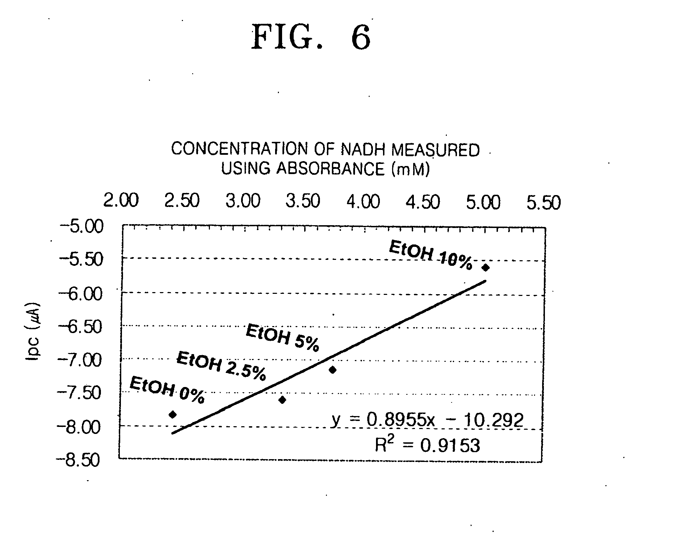

FIG. 6 is a graph illustrating a relationship between the value of a cathode peak current measured electrically and a NADH concentration measured optically. Referring to FIG. 6, electrical measurement and optical measurement are highly related.

According to the method of detecting the presence of toxic materials within a sample described in the present invention, toxicity of a compound can be detected by high sensitivity under a condition similar to a physiological condition.

## Claims

1. A method of detecting the presence of toxic materials within a sample, the method comprising:
contacting sub-mitochondria particles having competent mitochondrial enzyme formed from the inner membrane of mitochondria, an electron donor which transmits electrons to the electron transfer system of the sub-mitochondria particles, and a sample which will be tested;
adding tris-2,2'-bipyridyl-ruthenium (II) to the reaction mixture; and
measuring electrical variables of the reaction mixture.

2. The method of claim 1, wherein the sub-mitochondria particles are manufactured by sonicating whole mitochondria for crista membrane to reform to micelle.

3. The method of claim 1, wherein the mitochondrial enzyme is consisted of NADH-dehydrogenase, coenzyme-Q-cytochrome C reductase, and cytochrome C oxidase.

4. The method of claim 1. wherein the electron donor comprises at least one selected from the group consisting of NADH and NADPH.

5. The method of claim 1, wherein the electrical variables comprise at least one selected from the group consisting of current, voltage, impedance, and capacitance.

6. The method of claim 1, wherein the electrical variables are one of a cathode peak current and an anode peak current at cyclic voltammography.

7. The method of claim 6, wherein the current is measured at a voltage of 0.85 to 1.35 V.

8. A kit for electrically analyzing the presence of toxic materials within a sample, the kit comprising:
sub-mitochondria particles having competent mitochondrial enzyme formed from the inner membrane of mitochondria;
an electron donor which transmits electrons to the electron transfer system of the sub-mitochondria particles; and
tris-2,2'-bipyridyl-ruthenium (II).

9. The kit of claim 8, wherein the sub-mitochondria particles are manufactured by sonicating whole mitochondria for crista membrane to reform to micelle.

10. The kit of claim 8, wherein the sub-mitochondria particles are lyophilized.

11. The kit of claim 8, wherein the mitochondrial enzyme comprises at least one enzyme selected from the group consisting of NADH-dehydrogenase, coenzyme-Q-cytochrome C reductase, and cytochrome C oxidase.

12. The kit of claim 8, wherein the electron donor comprises at least one selected from the group consisting of NADH and NADPH.

## Patentansprüche

1. Verfahren zum Detektieren der Anwesenheit von toxischen Materialen in einer Probe, wobei das Verfahren umfasst:
In Kontakt bringen von Sub-Mitochondrienpartikeln, die ein kompetentes mitochondriales Enzym besitzen, gebildet aus der inneren Membran von Mitochondrien, einem Elektrondonor, der Elektronen zum Elektrontransfersystem der Sub-Mitochondrienpartikel überträgt, und einer Probe, die getestet wird;
Zugeben von Tris-2,2'-Bipyridyl-ruthenium (II) zum Reaktionsgemisch; und
Messen von elektrischen Variablen des Reaktionsgemisches.

2. Verfahren nach Anspruch 1, wobei die Sub-Mitochondrienpartikel durch das Beschallen von ganzen Mitochondrien, um Crista Membran zur Micelle umzuformen, hergestellt werden.

3. Verfahren nach Anspruch 1, wobei das mitochondriale Enzym aus NADH-Dehydrogenase, Coenzym-Q-Cytochrom C Reduktase, und Cytochrom C Oxidase besteht.

4. Verfahren nach Anspruch 1, wobei der Elektronendonor mindestens einen ausgewählt aus der Gruppe bestehend aus NADH und NADPH umfasst.

5. Verfahren nach Anspruch 1, wobei die elektrischen Variablen mindestens eine ausgewählt aus der Gruppe bestehend aus Strom, Spannung, lmpedanz und Kapazität umfasst.

6. Verfahren nach Anspruch 1, wobei die elektrischen Variablen eine von einem Kathoden Peak Strom und einem Anoden Peak Strom bei cyclischer Voltammographie sind.

7. Verfahren nach Anspruch 6, wobei der Strom bei einer Spannung von 0,85 bis 1,35 V gemessen wird.

8. Kit für das elektrische Analysieren der Anwesenheit von toxischen Materialien in einer Probe, wobei der Kit umfasst:
Sub-Mitochondrienpartikel, die ein kompetentes mitochondriales Enzym besitzen, gebildet aus der inneren Membran von Mitochondrien;
einen Elektronendonor, der Elektronen zum Elektronentransfersystem der Sub-Mitochondrienpartikel überträgt; und
Tris-2,2'-Bipyridyl-ruthenium (II).

9. Kit nach Anspruch 8, wobei die Sub-Mitochondrienpartikel durch Beschallen ganzer Mitochondrien, um Crista Membran zur Micelle umzuformen, hergestellt sind.

10. Kit nach Anspruch 8, wobei die Sub-Mitochondrienpartikel lyophilisiert sind.

11. Kit nach Anspruch 8, wobei das mitochondriale Enzym mindestens ein Enzym ausgewählt aus der Gruppe bestehend aus NADH-Dehydrogenase, Coenzym-Q-cytochrom C Reduktase, und Cytochrom C Oxidase umfasst.

12. Kit nach Anspruch 8, wobei der Elektronendonor mindestens einen ausgewählt aus der Gruppe bestehend aus NADH und NADPH umfasst.

## Revendications

1. Procédé de détection de la présence de matériaux toxiques dans un échantillon, ledit procédé consistant à :
mettre en contact des particules submitochondriales ayant une enzyme mitochondriale compétente formées à partir de la membrane interne des mitochondries, un donneur d'électrons qui transmet des électrons au système de transfert d'électrons des particules submitochondriales, et un échantillon à tester ;
ajouter du tris-2,2'-bipyridyl-ruthénium (II) au mélange réactionnel ; et
mesurer des variables électriques sur le mélange réactionnel.

2. Procédé selon la revendication 1, dans lequel les particules submitochondriales sont préparées par traitement de mitochondries entières aux ultrasons pour que la membrane interne mitochondriale se reforme en micelle.

3. Procédé selon la revendication 1, dans lequel l'enzyme mitochondriale consiste en NADH-déshydrogénase, coenzyme-Q-cytochrome C réductase, et cytochrome C oxydase.

4. Procédé selon la revendication 1, dans lequel le donneur d'électrons comprend au moins un élément choisi dans le groupe constitué du NADH et du NADPH.

5. Procédé selon la revendication 1, dans lequel les variables électriques comprennent au moins une variable choisie dans le groupe constitué du courant, de la tension, de l'impédance, et de la capacité.

6. Procédé selon la revendication 1, dans lequel les variables électriques consistent soit en un courant cathodique de crête soit en un courant anodique de crête, en voltammétrie cyclique.

7. Procédé selon la revendication 6, dans lequel le courant est mesuré à une tension de 0,85 à 1,35 V.

8. Trousse permettant l'analyse électrique de la présence de matériaux toxiques dans un échantillon, ladite trousse comprenant :
des particules submitochondriales ayant une enzyme mitochondriale compétente formées à partir de la membrane interne des mitochondries ;
un donneur d'électrons qui transmet des électrons au système de transfert d'électrons des particules submitochondriales ; et
du tris-2,2'-bipyridyl-ruthénium (II).

9. Trousse selon la revendication 8, dans laquelle les particules submitochondriales sont préparées par traitement de mitochondries entières aux ultrasons pour que la membrane interne mitochondriale se reforme en micelle.

10. Trousse selon la revendication 8, dans laquelle les particules submitochondriales sont lyophilisées.

11. Trousse selon la revendication 8, dans laquelle l'enzyme mitochondriale comprend au moins une enzyme choisie dans le groupe constitué de la NADH-déshydrogénase, de la coenzyme-Q-cytochrome C réductase, et de la cytochrome C oxydase.

12. Trousse selon la revendication 8, dans laquelle le donneur d'électrons comprend au moins un élément choisi dans le groupe constitué du NADH et du NADPH.
